# EUROPEAN PATENT SPECIFICATION

(11) **EP 3 698 763 B1**
(45) Date of publication and mention of the grant of the patent: **17.08.2022**
(21) Application number: 18897680.7
(22) Date of filing: 07.12.2018
(51) Int. Cl.: A61F 13/49

(54) **UNDERPANTS-SHAPED ABSORBENT ARTICLE**
UNTERHOSENFÖRMIGER ABSORBIERENDER ARTIKEL
ARTICLE ABSORBANT EN FORME DE CULOTTE

(30) Priority: 28.12.2017 JP 2017254955
(43) Date of publication of application: 26.08.2020
(73) Proprietor: Unicharm Corporation, Ehime 799-0111 (JP)
(72) Inventor: MAKI, Hideaki, Kanonji-shi, Kagawa 769-1602 (JP); INOUE, Takuya, Kanonji-shi, Kagawa 769-1602 (JP); OKUBO, Tetsuo, Kanonji-shi, Kagawa 769-1602 (JP); SHIMIZU, Noriko, Kanonji-shi, Kagawa 769-1602 (JP)
(74) Representative: Dolleymores
(86) International application number: PCT/JP2018/045077
(87) International publication number: WO 2019/131060

(56) References cited:
- WO-A1-2016/159095
- JP-A- 2002 045 399
- JP-A- 2002 065 731
- JP-A- 2015 073 637
- JP-U- H0 484 309
- US-A1- 2006 254 708
- US-A1- 2012 226 255

## Description

### [Technical Field]

The present invention relates to an underpants-shaped absorbent article.

### [Background Art]

Pull-on disposable diapers are known as an example of an underpants-shaped absorbent article. PTL 1 discloses a pull-on disposable diaper in which an absorbent main body is provided spanning between a front-panel exterior body and a back-panel exterior body, and the two widthwise side edges of the front-panel exterior body and the back-panel exterior body are joined to each other so as to form a waist opening. In the front-panel exterior body and the back-panel exterior body, waist elastic members are fixed stretching between an inner sheet and an outer sheet (non-stretchy sheets).

### [Citation List]

### [Patent Literature]

[PTL 1] Japanese Patent Application Publication No. 2013-138795

US 2006/0254708 A1 relates to a method for manufacturing a disposable wearing article, comprising: a step of manufacturing an elastic laminated body by laminating two webs while inserting an elastic member in an extended state in a web length direction in between; a step of cutting the elastic laminated body in a length direction so that a concave portion and a convex portion appear alternately; a step of attaching a cover sheet to bridge between the concave portion and the convex portion of cut first elastic laminated body and second elastic laminated body, respectively; a step of widening the first elastic laminated body and the second elastic laminated body to which the cover sheet is attached; and a step of attaching an absorber onto the cover sheet.

JP 2002-45399 relates to a method for making a pants-type disposable diaper. An elastic part around the waist is adhered between an outside sheet and a skin side sheet only in the part where right and left side edges around the waist are connected and in neighbouring side positions. The outside sheet and the skin side sheet are adhered to each other without adhering the elastic part around the waist therebetween at least at the part where an absorber is positioned.

US 2012/0226255 A1 discloses a diaper having a chassis comprising a front waist region, a rear waist region and a crotch region extending between the front and rear waist regions: a liquid-absorbent structure extends across the crotch region into the front and rear waist regions; elastic sheets are respectively provided in the front and rear waist regions to be contractible in the transverse direction.

### [Summary of Invention]

### [Technical Problem]

In a pull-on disposable diaper that is provided with elastic members such as in PTL 1, the stretchability of the elastic members arranged around the waist allows the front-panel (back-panel) exterior body to fit along the wearer's waist, and also allows the upper end portion of the absorbent main body to fit to the wearer's lower stomach region.

However, if string-like elastic members are used as the waist elastic members, linear marks are likely to be formed in the wearer's skin due to constriction by the elastic force of the string-like elastic members. In particular, if the wearer is an infant who has sensitive skin, for example, there is a risk of poor texture in the stomach region or the like, thus causing an increased level of discomfort.

Also, with the diaper in PTL 1, the waist elastic members provide fit at both the front-panel (back-panel) exterior body and the upper end portion of the absorbent main body at the same time, and therefore there is a risk that these members will easily shift in position when the wearer moves their body. For example, when the wearer moves their legs back and forth, there are cases where the absorbent main body moves up and down (forward and backward) on the stomach side of the wearer in accordance with the leg movement, and the front-panel exterior body moves in conjunction with the movement of the absorbent main body. In such a case, there is a risk that the front-panel exterior body will shift in position, thus leading to the risk of a poor fit and the leakage of excrement from a gap that forms in the waist portion.

The present invention was achieved in light of conventional problems such as that described above, and an aspect of the present invention is to provide an underpants-shaped absorbent article that has a favorable texture and enables the fit to be maintained even when body movement occurs.

### [Solution to Problem]

The present invention provides the underpants-shaped absorbent article of independent claim 1. The dependent claims specify preferred but optional features.

### [Advantageous Effects of Invention]

According to the present invention, it is possible to provide an underpants-shaped absorbent article that has a favorable texture and enables the fit to be maintained even when body movement occurs.

### [Brief Description of the Drawings]

FIG. 1 is a perspective view of a pull-on disposable diaper 1 (hereinafter, "diaper").
FIG. 2 is a plan view of the diaper 1 in an unfolded and stretched state.
FIG. 3 is a cross-sectional view taken along a line I-I in FIG. 2.
FIG. 4 is an enlarged view of a front waist portion 20 (first waist portion) and an absorbent main body 10 in FIG. 3.
FIGS. 5A and 5B are schematic cross-sectional views illustrating details of the fit of the diaper 1 on the wearer's stomach side.
FIG. 6 is a schematic cross-sectional view illustrating a variation of a method for fixing a stretchy sheet 24 of the front waist portion 20.

### [Description of Embodiments]

At least the following matters will become clear with the description of this specification and the attached drawings.

An underpants-shaped absorbent article having an up-down direction, a left-right direction, and a front-rear direction that intersect each other,
the underpants-shaped absorbent article including:
an absorbent main body; and
a first waist portion that is joined to the absorbent main body and that includes a stretchy sheet having stretchability in the left-right direction,
   the stretchy sheet having a lower end,
   the lower end being located at a position separated upward in the up-down direction by a predetermined distance from an upper end of the absorbent main body.

According to this underpants-shaped absorbent article, the region provided with the stretchy sheet in the first waist portion fits in surface contact with the wearer's skin, thus making it possible to realize a favorable texture. Also, the stretchy sheet and the absorbent main body are not overlapped with each other in the up-down direction, thus enabling these members to independently follow movement of the wearer's body without moving in coordination with each other, and therefore a favorable fit can be realized.

In such a underpants-shaped absorbent article,
the first waist portion includes a plurality of elastic members having stretchability in the left-right direction, and
left-right-direction mean stress in an up-down-direction region of the first waist portion that is between the lower end of the stretchy sheet and an elastic member located at an uppermost position among the plurality of elastic members
   is smaller than
a left-right-direction mean stress in an up-down-direction region of the first waist portion in which the stretchy sheet is arranged.

According to this underpants-shaped absorbent article, the first waist portion can easily deform freely in the region between the lower end of the stretchy sheet and the elastic members when the absorbent article is put on. Also, in the region where the stretchy sheet is arranged, the first waist portion can easily fit to the wearer's waist.

In such a underpants-shaped absorbent article, it is desirable that the elastic members are arranged in at least a portion of an up-down-direction region of the first waist portion that is overlapped with the absorbent main body.

According to this underpants-shaped absorbent article, contractive force of the elastic members acts on the absorbent main body, thus allowing the absorbent main body to fit to the wearer's body, and making it possible to suppress positional shifting.

In such a underpants-shaped absorbent article, it is desirable that the elastic members are not arranged in an up-down-direction region of the first waist portion in which the stretchy sheet and the absorbent main body are not overlapped.

According to this underpants-shaped absorbent article, the stretchability of the elastic members (string-like elastic members) is not likely to act in the region in the up-down direction between the stretchy sheet and the absorbent main body (in the buffer region), and therefore the first waist portion can easily deform flexibly in this region. Accordingly, the absorbent main body and the region provided with the stretchy sheet are likely to independently follow movement of the wearer's body, and it is possible to further improve the fit of the underpants-shaped absorbent article.

In such a underpants-shaped absorbent article, it is desirable
that the first waist portion includes a plurality of the elastic members, and
that the left-right-direction mean stress in the up-down-direction region of the first waist portion in which the stretchy sheet is arranged is smaller than
   a left-right-direction mean stress in an up-down-direction region of the first waist portion in which the plurality of elastic members are arranged.

According to this underpants-shaped absorbent article, when the absorbent article is put on, the first waist portion does not excessively constrict the wearer's waist, and the wearer is not likely to feel discomfort. On the other hand, a relatively large amount of constriction force acts in the absorbent main body, and therefore even if the absorbent main body is pulled or subjected to a large amount of external force, positional shifting of the absorbent main body is not likely to occur.

In such a underpants-shaped absorbent article, it is desirable
that the first waist portion includes a plurality of the elastic members, and
that concerning an up-down-direction region of the first waist portion in which the plurality of elastic members are arranged,
   a left-right-direction mean stress in a region that is above a center in the up-down direction is smaller than a left-right-direction mean stress in a region that is below the center in the up-down direction.

According to this underpants-shaped absorbent article, the amount of stress decreases in stages from the lower side of the absorbent main body toward the upper side (upper end portion). In other words, the amount of stress decreases the closer the position is to the region between the lower end of the stretchy sheet and the elastic member (buffer region) . This makes it possible to more effectively buffer force acting in the regions above and below this region.

In such a underpants-shaped absorbent article, it is desirable
that the absorbent main body includes an absorbent body that absorbs an excreted fluid, and
that a left-right-direction mean stress in an up-down-direction region of the first waist portion that is between the elastic member located uppermost and an upper end of the absorbent body
   is smaller than
   a left-right-direction mean stress in an up-down-direction region of the first waist portion that is between the upper end of the absorbent body and an up-down-direction center of the up-down-direction region in which the plurality of elastic members are arranged.

According to this underpants-shaped absorbent article, the amount of stress can be set lower the closer the position is to the upper end portion of the absorbent main body. Furthermore, in the region overlapped with the absorbent body, the absorbent body can easily fit to the wearer's body. On the other hand, in the region not overlapped with the absorbent body, excessive constriction does not occur, and a soft texture can be realized.

In such a underpants-shaped absorbent article, the first waist portion includes a plurality of the elastic members, and
an up-down-direction length of the up-down-direction region in which the stretchy sheet is arranged
   is longer than
an up-down-direction length between the lower end of the stretchy sheet and the elastic member located uppermost.

According to this underpants-shaped absorbent article, in the region in which the stretchy sheet is arranged, a larger percentage of the first waist portion fits in surface contact with the wearer's skin, thus making it possible to suppress positional shifting of the first waist portion.

In such a underpants-shaped absorbent article, it is desirable
that the absorbent main body includes an absorbent body that absorbs an excreted fluid,
that the first waist portion includes a plurality of the elastic members, and
that an up-down-direction length between the upper end of the absorbent main body and an upper end of the absorbent body
   is longer than
an up-down-direction length between the lower end of the stretchy sheet and the elastic member located uppermost.

According to this underpants-shaped absorbent article, the upper end portion of the absorbent main body is more likely to fit to the wearer's stomach region, thus making it more likely to suppress positional shifting of the absorbent main body.

In such a underpants-shaped absorbent article, it is desirable
that the underpants-shaped absorbent article further comprises:
   a non-stretchy sheet that constitutes the first waist portion; and
   a fixing portion that fixes the stretchy sheet to the non-stretchy sheet,
that the stretchy sheet has a lower end portion in the up-down direction, and
that the lower end portion of the stretchy sheet includes a folded portion that has been folded upward at a lower end of the fixing portion.

According to this underpants-shaped absorbent article, the stretchy sheet is overlaid on itself in the folded portion, thus increasing the amount of stretching force, which suppresses positional shifting of the front waist portion. Also, the position of the lower end portion of the stretchy sheet can be changed by changing the position of the lower end of the fixing portion, thus making it possible to freely adjust the size of the region in which the stretchy sheet and the absorbent main body are not overlapped (buffer region) . This makes it more likely to realize an absorbent article that has optimal fit in accordance with the target wearer's physique or the like.

### Embodiment

The following describes an embodiment of an underpants-shaped absorbent article according to the present invention by way of example of a disposable diaper for infants. Note that the underpants-shaped absorbent article according to the present invention is not limited to being a disposable diaper for infants, and is also applicable to a disposable diaper for adults and sanitary shorts, for example.

### Configuration of pull-on disposable diaper 1

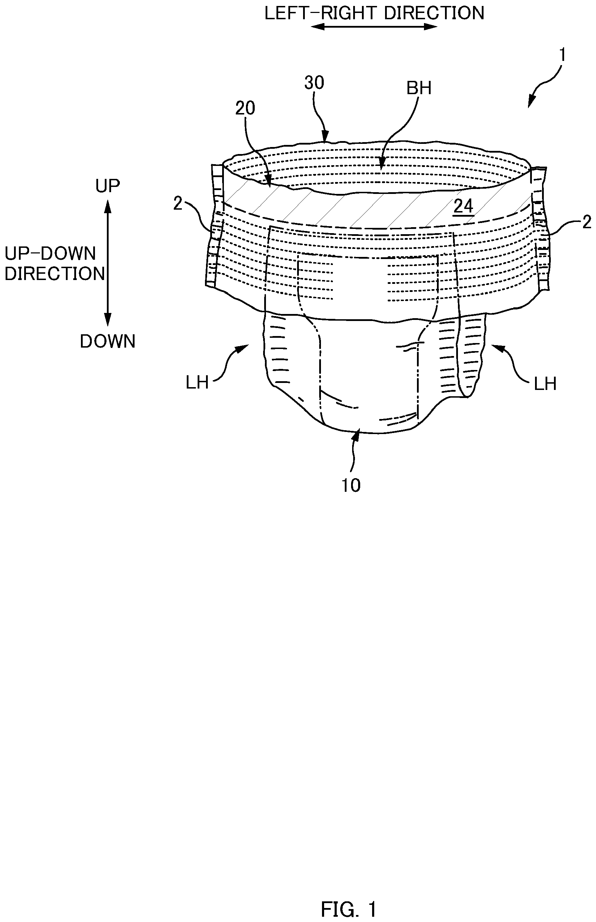

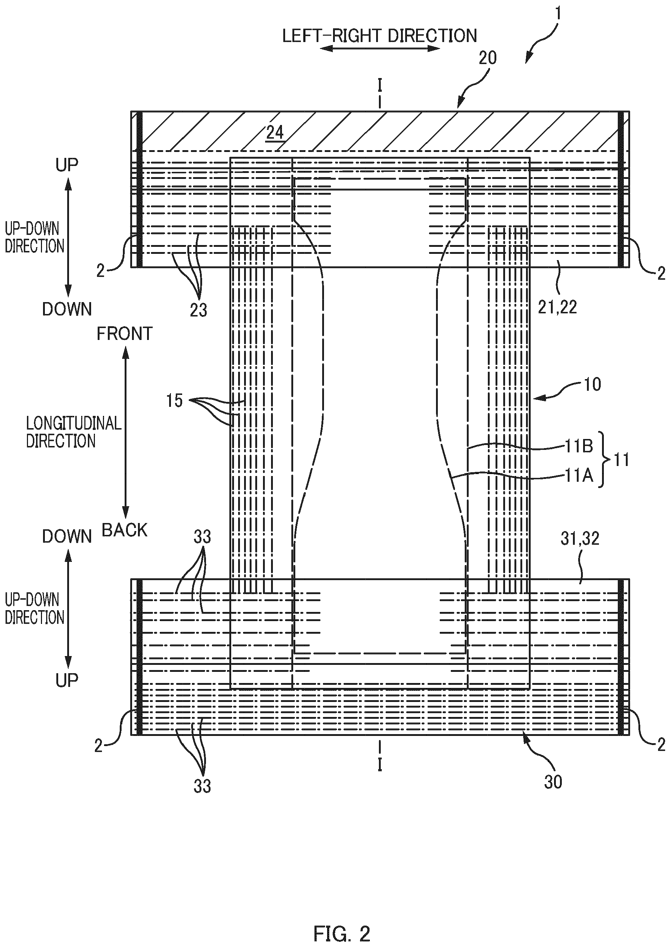

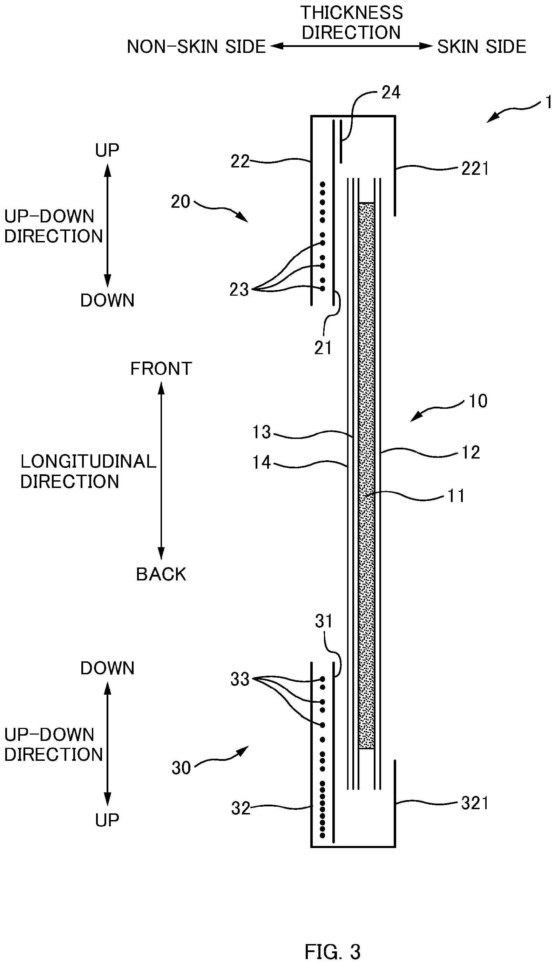

FIG. 1 is a perspective view of a pull-on disposable diaper 1 (hereinafter, "diaper"). FIG. 2 is a plan view of the diaper 1 in an unfolded and stretched state. FIG. 3 is a cross-sectional view taken along a line I-I in FIG. 2.

In the underpants-shaped state shown in FIG. 1, the diaper 1 has an up-down direction and a left-right direction, and includes a waist opening BH and a pair of leg openings LH. With respect to the up-down direction, the waist opening BH side is the upper side, and the crotch side is the lower side. Also, as shown in FIG. 3, the thickness direction refers to the direction in which constituent members of the diaper 1 are overlaid on each other, the skin side refers to the side that comes into contact with the wearer in the thickness direction, and the non-skin side refers to the side that does not come into contact with the wearer in the thickness direction.

The diaper 1 also includes an absorbent main body 10, a front waist portion 20, and a back waist portion 30, which are three members that are rectangular in a plan view. The front waist portion 20 (first waist portion) comes into contact with the front region of the wearer, and the back waist portion 30 (second waist portion) comes into contact with the back region of the wearer.

When the diaper 1 is in the unfolded state shown in FIG. 2, the front waist portion 20 and the back waist portion 30 are arranged such that the longitudinal directions thereof conform to the left-right direction of the diaper 1. The end portion of the absorbent main body 10 on the longitudinal one side thereof is arranged in the central portion of the front waist portion 20 with respect to the left-right direction, and the end portion of the absorbent main body 10 on the longitudinal other side thereof is arranged in the central portion of the back waist portion 30 with respect to the left-right direction.

When the diaper 1 is in the unfolded state shown in FIG. 2, the absorbent main body 10 is folded one time at approximately the longitudinal center such that the longitudinal direction of the absorbent main body 10 conforms to the up-down direction of the diaper 1. The two side end portions of the front waist portion 20 in the left-right direction of the diaper 1 are joined to the two side end portions of the back waist portion 30 with use of heat welding, ultrasonic welding, or the like to form a pair of joining regions 2, thus obtaining the diaper 1 in the underpants-shaped state shown in FIG. 1.

As shown in FIG. 3, the absorbent main body 10 includes an absorbent body 11, a liquid-permeable top sheet 12 arranged on the skin side of the absorbent body 11, a liquid-impermeable back sheet 13 arranged on the non-skin side of the absorbent body 11, and an exterior sheet 14 arranged on the non-skin side of the back sheet 13.

The absorbent body 11 includes an absorbent core 11A that absorbs and holds an excreted fluid such as urine, and a liquid-permeable core-wrapping sheet 11B that covers the absorbent core 11A. For example, the absorbent core 11A is constituted by liquid-absorbent fibers such as pulp containing a superabsorbent polymer (SAP), which are molded into a predetermined shape.

Also, as shown in FIG. 2, leg elastic members 15 are provided in two side portions of the absorbent main body 10 in the left-right direction, thus allowing the diaper 1 to fit to the wearer's legs. For example, the leg elastic members 15 are fixed in a state of being stretched in the longitudinal direction of the absorbent main body 10 between two layers of the exterior sheet 14 that has been folded inward in the left-right direction. The leg elastic members 15 are string-like elastic members, stretchy sheets, or the like.

As shown in FIG. 3, the front waist portion 20 and the back waist portion 30 respectively include inner layer sheets 21 and 31, outer layer sheets 22 and 32 overlaid on the inner layer sheets 21 and 31, and multiple string-like elastic members 23 and 33.

The outer layer sheets 22 and 32 are arranged on the non-skin side of the inner layer sheets 21 and 31. The inner layer sheets 21 and 31 and the outer layer sheets 22 and 32 are arranged on the non-skin side of the absorbent main body 10. It should be noted that upper end portions 221 and 321 of the outer layer sheets 22 and 32 are folded downward so as to cover the longitudinal end portions of the absorbent main body 10 from the skin side.

The inner layer sheets 21 and 31 and the outer layer sheets 22 and 32 are each a non-stretchy sheet that has substantially no stretchability in the left-right direction of the diaper 1. The inner layer sheets 21 and 31 and the outer layer sheets 22 and 32 can be constituted by an SMS nonwoven fabric sheet (spunbond/meltblown/spunbond nonwoven fabric sheet), a spunbond nonwoven fabric sheet, an air-through nonwoven fabric sheet, a plastic sheet, a porous plastic sheet, or a laminate sheet including layers of any of the same.

The string-like elastic members 23 and 33 are arranged side-by-side in the up-down direction between the inner layer sheets 21 and 31 and the outer layer sheets 22 and 32, and are fixed in a state of being stretched in the left-right direction. The front waist portion 20 and the back waist portion 30 can thus stretch and contract in the left-right direction so as to fit to the wearer's waist. The string-like elastic members 23 and 33 can be constituted by a string-like elastic member made of rubber, spandex, or the like.

In the back waist portion 30, the string-like elastic members 33 are arranged within a range from the upper end portion to the lower end portion. However, the upper end portion of the front waist portion 20 is not provided with the string-like elastic members 23, and instead is provided with a stretchy sheet 24 that has stretchability in the left-right direction.

According to this configuration in which the upper end portion of the front waist portion 20 is not provided with the string-like elastic members 23 but is provided with the stretchy sheet 24, the upper end portion of the front waist portion 20 comes into close surface contact with the wearer. This therefore suppresses localized constriction, and can suppress the formation of marks by elastic members. In particular, an infant has a bulging stomach region, and therefore it is preferable to suppress constriction in the front waist portion 20.

Also, stretching force in the upper end portions of the waist portions 20 and 30 largely influences the ability to easily spread open the waist opening BH when the diaper 1 is to be put on. The stretchy sheet 24 has a characteristic of easily stretching from the natural state, but not easily contracting from the stretched state. For this reason, by providing the stretchy sheet 24 in the upper end portion of the front waist portion 20, it is possible to make it easier to spread open the waist opening BH when putting on the diaper 1, and also prevent constriction of the front region after the waist portions 20 and 30 have been fitted to the wearer.

The stretchy sheet 24 can be constituted by a stretchy-fiber nonwoven-fabric sheet that is manufactured by any of various known manufacturing methods, such as a spunbond nonwoven fabric sheet, an air-through nonwoven fabric sheet, a needle punch nonwoven fabric sheet, and the like, which are constituted by elastic fibers having a mass of 10 to 40 g/m², or more preferably 15 to 30 g/m². The raw material constituting the elastic fibers is a thermoplastic elastomer, rubber, or the like. Particularly in the case where the raw material is a thermoplastic elastomer, it is possible to perform melt spinning with use of an extruder similarly to normal thermoplastic resin. The thus-obtained fibers can be easily heat welded, which is favorable for a stretchy-fiber nonwoven-fabric sheet. Examples of thermoplastic elastomers include styrene elastomer, olefin elastomer, polyester elastomer, and polyurethane elastomer. It is possible to use any of the above on their own, or a combination of two or more. The stretchy sheet need only exhibit elasticity in at least the left-right direction, and may exhibit elasticity in two or more directions. The stretch factor of the stretchy sheet 24 in the left-right direction is preferably a factor of approximately 1.2 to 3.0.

### Fit of diaper 1 on stomach side

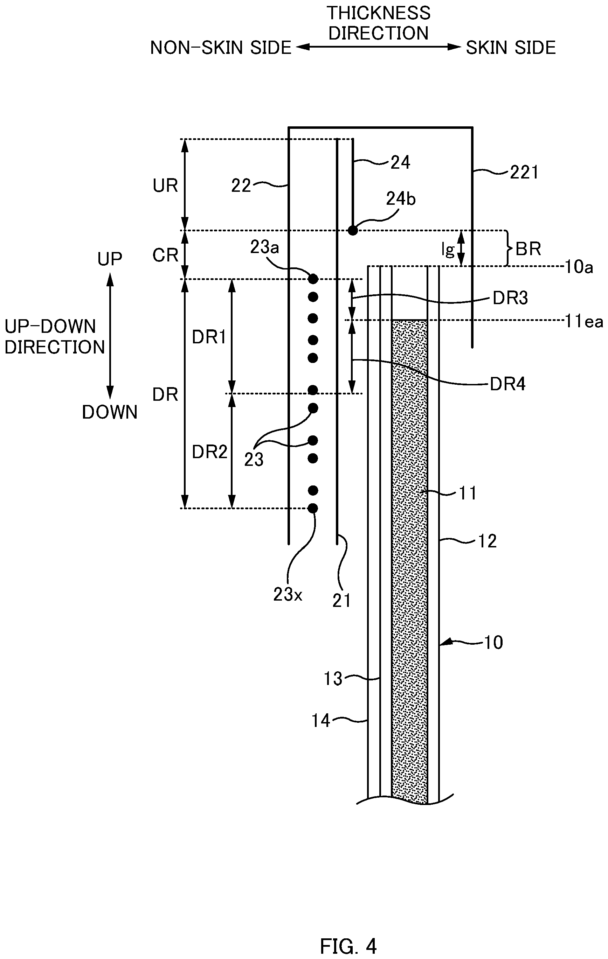

The following describes the fit of the diaper 1 on the wearer's stomach side when the diaper 1 is put on. FIG. 4 is an enlarged view of the front waist portion 20 (first waist portion) and the absorbent main body 10 in FIG. 3.

As previously described, the front waist portion 20 includes the inner layer sheet 21, the outer layer sheet 22, and the stretchy sheet 24. In an intermediate state during manufacturing, the outer layer sheet 22, the inner layer sheet 21, and the stretchy sheet 24 are overlaid on each other in this order beginning from the non-skin side in the thickness direction. The stretchy sheet 24 has a lower end 24b that extends in the left-right direction.

In the diaper 1 of the present embodiment, the lower end 24b of the stretchy sheet 24 is arranged at a position that is separated by a distance lg upward from an upper end 10a of the absorbent main body 10. In other words, a gap lg is provided in the up-down direction between the lower end 24b of the stretchy sheet 24 and the upper end 10a of the absorbent main body 10. The region corresponding to the gap lg in the up-down direction will be called a "buffer region BR". As shown in FIG. 4, the buffer region BR is a region that has fewer sheet members overlaid in the thickness direction, and has the lowest stiffness in the front waist portion 20.

Also, the up-down-direction region of the front waist portion 20 where the stretchy sheet 24 is arranged will be called an "upward region UR". The front waist portion 20 can be fitted to the wearer's waist by left-right-direction contraction of the stretchy sheet 24 in the upward region UR when the diaper 1 is put on.

Also, in the front waist portion 20, at least a portion of the up-down-direction region where the string-like elastic members 23 are arranged is overlapped with the upper end portion of the absorbent main body 10. The region in the up-down direction between a string-like elastic member 23a arranged at the uppermost position and a string-like elastic member 23x arranged at the lowermost position will be called a "downward region DR". The absorbent main body 10 and the front waist portion 20 can be fitted to the wearer's body by left-right-direction contraction of the string-like elastic members 23 in the downward region DR when the diaper 1 is put on. In particular, the upper end portion of the absorbent main body 10 is pressed toward the wearer's skin due to contraction of the string-like elastic members 23, thus suppressing positional shifting when the diaper 1 is put on.

Also, the region between the upward region UR and the downward region DR in the up-down direction will be called a "middle region CR". Specifically, the middle region CR is the region where neither the stretchy sheet 24 nor the string-like elastic members 23 are provided, and no stretchability in the left-right direction is exhibited by such elastic members. Note that in FIG. 4, the buffer region BR is included in the middle region CR.

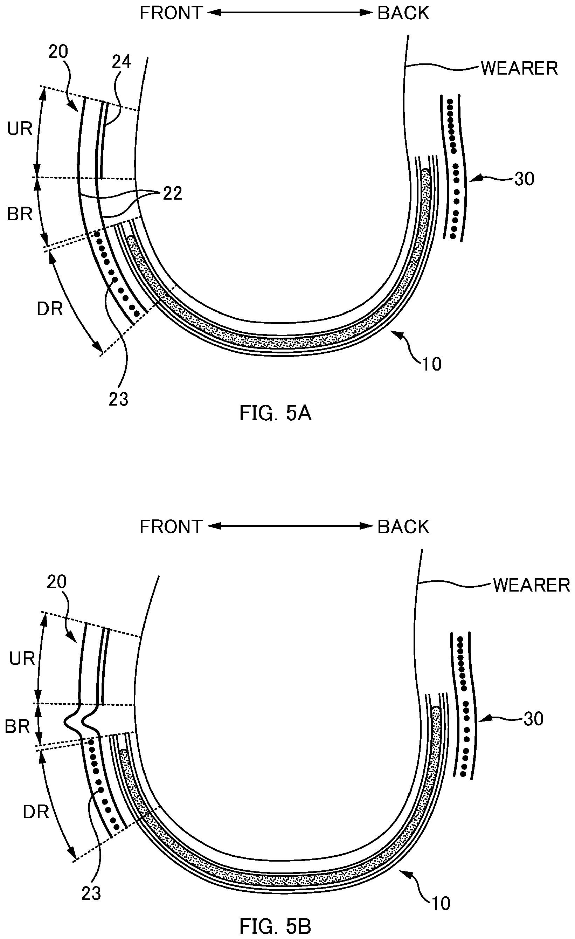

FIGS. 5A and 5B are schematic cross-sectional views illustrating details of the fit of the diaper 1 on the wearer's stomach side. FIG. 5A shows a cross-section taken in the left-right direction in the case where the wearer is in a normal posture with a straight spine. FIG. 5B shows a cross-section taken in the left-right direction in the case where the wearer is in a forward-bent posture, such as when sitting in a slouched manner.

In the normal posture in FIG. 5A, due to contractive force exhibited by the stretchy sheet 24 in the upward region UR, the upper end portion of the front waist portion 20 fits in surface contact with the bulging portion of the wearer's stomach region. Due to this fitting of the upper end portion of the front waist portion 20, the upper end portion acts as a belt, thus suppressing the case where the front waist portion 20 shifts away from the wearer's stomach region. Also, due to the upward region UR fitting in surface contact, constriction pressure does not become locally concentrated on the wearer's skin, and such pressure is distributed over the entirety of the stretchy sheet 24. Accordingly, the wearer is not likely to feel discomfort, and it is possible to suppress the case where a mark remains in the stomach region due to constriction by elastic members.

On the other hand, due to the contractive force exhibited by the string-like elastic members 23 in the downward region DR, the upper end portion of the absorbent main body 10 fits to the region below the bulge of the wearer's stomach region. This region is inclined diagonally downward from the peak of the stomach region bulge toward the crotch region, and therefore by allowing each of the string-like elastic members 23 to exhibit contractive force to increase the amount of constriction, it is possible to suppress the case where the absorbent main body 10 shifts downward, and the case where a gap forms between the upper end portion of the absorbent main body 10 and the wearer's skin. Note that as shown in FIG. 4, more members (e.g., the inner layer sheet 21 and the absorbent body 11) are overlaid on the skin side of the string-like elastic member 23 in the downward region DR, and therefore even if the constriction force is increased in this region, a constriction mark due to the string-like elastic members 23 is not likely to be formed on the wearer's skin.

Also, the front waist portion 20 fits to the wearer's stomach region in the buffer region BR (middle region CR) between the upward region UR and the downward region DR. It should be noted that elastic members such as the stretchy sheet 24 are not provided in the buffer region BR, thus making it unlikely for stretchability to act in the left-right direction in the buffer region BR. The front waist portion 20 therefore does not necessarily need to be in close contact with the wearer's skin in the buffer region BR.

When the wearer in the state shown in FIG. 5A then shifts to a forward-bent posture, the wearer's stomach region changes to a state of being smaller in the up-down direction. At this time, the stretchy sheet 24 continues to exhibit stretching force in the upward region UR, thus maintaining the fit of the front waist portion 20 in this region. Similarly, the string-like elastic members 23 continue to exhibit stretching force in the downward region DR, thus maintaining the fit in the upper end portion of the absorbent main body 10 in this region. Accordingly, the distance in the up-down direction between the position of the upward region UR and the position of the downward region DR decreases in the wearer's stomach region.

On the other hand, in the buffer region BR, there are fewer overlaid sheet members and the stiffness is lower, and therefore due to being compressed vertically by the upward region UR and the downward region DR, the front waist portion 20 separates from the wearer's skin (stomach region) in the buffer region BR so as to deform in manner of protruding toward the non-skin side as shown in FIG. 5B. This deformation of the buffer region BR suppresses the case where the upward region UR and the downward region DR collide and the fit degrades. In other words, even if the positions of the upward region UR and the downward region DR change relative to each other due to movement of the wearer's body, it is possible to maintain the fit in these regions UR and DR.

In contrast to the case in FIG. 5B, if the wearer shifts to a backward arched posture such as when stretching their back, the wearer's stomach region will become stretched in the up-down direction, and therefore the upward region UR will move upward and the downward region DR will move downward. Even in this case, the buffer region BR deforms so as to stretch upward and downward, thus making it possible to maintain the fit in both the upward region UR and the downward region DR.

Also, even in the case where the absorbent main body 10 sags downward due to absorbing excrement such as urine and becoming heavier, or the absorbent main body 10 is pulled downward by movement of the wearer's legs, the existence of the buffer region BR makes it unlikely for the force pulling the absorbent main body 10 downward to reach the upward region UR. Accordingly, the front waist portion 20 is not likely to become positionally shifted, and it is possible to make it unlikely for the wearer to feel unpleasantness or discomfort.

If the lower end 24b of the stretchy sheet 24 overlaps the front-side upper end portion of the absorbent main body 10, that is to say if the upward region UR and the downward region DR are overlapped with each other in the up-down direction and the buffer region is not formed, then in a case where the position of the downward region DR shifts due to the absorbent main body 10 being pulled for example, the position of the upward region UR will also correspondingly shift, and the fit will degrade. In contrast, in the diaper 1 of the present embodiment, the buffer region BR is provided between the lower end 24b of the stretchy sheet 24 and the upper end 10a of the absorbent main body 10 in the up-down direction, thus allowing the upward region UR and the downward region DR to independently follow movement of the wearer's body. This therefore makes it possible to maintain the fit of the front waist portion 20 and the absorbent main body 10.

Also, in the present embodiment, the string-like elastic members 23 (elastic members) are provided below the upper end 10a of the absorbent main body 10. In other words, the string-like elastic members 23 are not provided in the buffer region BR that is between the lower end 24b of the stretchy sheet 24 and the upper end 10a of the absorbent main body 10 in the up-down direction. That is to say, the string-like elastic members 23 are not overlapped with the buffer region BR in the up-down direction, and stretchability is not likely to act in the buffer region BR due to the string-like elastic members 23. Accordingly, the front waist portion 20 is more likely to flexibly deform in the buffer region BR, positional shifting of the upward region UR and the downward region DR is even less likely to occur, and it is possible to further improve the fit when the diaper 1 is put on. It should be noted that even if the buffer region BR and the string-like elastic members 23 are arranged overlapping each other in the up-down direction, as long as the stretchy sheet 24 and the upper end 10a of the absorbent main body 10 are separated from each other in the up-down direction, it is possible to realize the "buffer" function of the buffer region BR.

Also, in the diaper 1 of the present embodiment, the left-right-direction mean stress σ_{BR} in the buffer region BR of the front waist portion 20 is smaller than the left-right-direction mean stress σ_{UR} in the upward region UR of the waist portion 20 (σ_{BR} < σ_{UR}). Accordingly, when the diaper 1 is put on, the front waist portion 20 is likely to fit to the wearer's waist in the upward region UR, and the front waist portion 20 is likely to deform freely in the buffer region BR. Accordingly, the downward region DR and the upward region UR are even less likely to move in coordination due to deformation of the buffer region BR, and positional shifting and the like of the front waist portion 20 is not likely to occur at the wearer's waist.

Note that the aforementioned stress in the buffer region BR and the upward region UR can be measured by performing a stress test such as follows, for example. First, the joining regions 2 formed at the two side portions of the pull-on diaper 1 are cut away, and the front waist portion 20 and the back waist portion 30 are separated from each other. Measurement target test pieces are then respectively cut out from the buffer region BR and the upward region UR of the front waist portion 20. For each test piece, a section of the test piece having a length of 140 mm in the left-right direction is clamped, as a measurement section, between the chucks of a tensile tester (Instron Co.). Then the test piece is then pulled on both sides in the left-right direction at the speed of 100 mm/min until it has stretched to a length of approximately 270 mm, and then the test piece is allowed to contract at the speed of 100 mm/min until the chuck separation distance returns to 140 mm. This cycle is repeated two times, and when the test piece has been stretched to a predetermined length in the second cycle, the tensile load is measured, and the stress is calculated.

Also, the left-right-direction mean stress σ_{UR} acting on the front waist portion 20 in the upward region UR is smaller than the left-right-direction mean stress σ_{DR} acting on the absorbent main body 10 in the downward region DR (σ_{UR} < σ_{DR}). Accordingly, when the diaper 1 is put on, the front waist portion 20 does not excessively constrict the stomach side of the wearer's waist, and the wearer is not likely to feel discomfort. On the other hand, a relatively large amount of force acts in the upper end portion of the absorbent main body 10, and therefore even if the absorbent main body 10 is forcefully pulled downward due to movement of the wearer's legs or the like, positional shifting of the absorbent main body 10 is not likely to occur.

Also, a configuration is possible in which in the front waist portion 20, the string-like elastic members 23 are arranged so as to have different stretch factors depending on their position in the up-down direction, and therefore the amount of stress acting in the downward region DR differs depending on the position in the up-down direction. Specifically, it is desirable that the amount of force acting in the absorbent main body 10 decreases the closer the position is to the buffer region BR. This is because if the amount of stress is high in the upper end portion of the absorbent main body 10, which is adjacent to the buffer region BR, there is a risk that the action of force cannot be entirety buffered in the buffer region BR, and positional shifting of the front waist portion 20 will occur in conjunction with movement of the absorbent main body 10.

Accordingly, it is desirable that the left-right-direction mean stress σ_{DR1} in a region DR1 that is above the vertical center in the downward region DR is smaller than the left-right-direction mean stress σ_{DR2} in a region DR2 that is below the vertical center in the downward region DR (σ_{DR1} < σ_{DR2}). According to this configuration, the amount of stress decreases in stages from the lower side of the absorbent main body 10 toward the upper side (upper end portion), and therefore the amount of stress is lower in the vicinity of the buffer region BR, and it is possible to more effectively buffer force that acts in the upward region UR and force that acts in the downward region DR.

Furthermore, in the up-down direction, concerning a region DR3 which is a part of the aforementioned region DR1 that is not overlapped with the absorbent body 11 and concerning a region DR4 which is a part of the aforementioned region DR1 that is overlapped with the absorbent body 11, it is desirable that the left-right-direction mean stress σ_{DR3} in the region DR3 is smaller than the left-right-direction mean stress σ_{DR4} in the region DR4 (σ_{DR3} < σ_{DR4}). According to this configuration, the amount of stress decreases in stages from the lower side of the absorbent main body 10 toward the upper end portion, and the amount of stress is high in the region DR4 that is overlapped with the absorbent body 11, thus allowing the absorbent body 11 to more easily fit to the wearer's body. On the other hand, in the region DR3 that is not overlapped with the absorbent body 11, the number of sheet member layers is lower, and the absorbent main body 10 has a lower stiffness, and therefore the amount of stress is reduced in the region DR3, thus making it possible to prevent excessive constriction in the upper end portion of the absorbent main body 10 so as to achieve a soft texture.

Also, in the up-down-direction, the length of the region in which the stretchy sheet 24 is arranged (the upward region UR) is longer than the length of the region between the lower end 24b of the stretchy sheet 24 and the string-like elastic member 23a located uppermost (the middle region CR). Due to the up-down-direction length of the upward region UR being longer than the up-down-direction length of the middle region CR, it is possible to increase the percentage of the portion of the front waist portion 20 that fits in surface contact with the wearer's skin in the upward region UR, and it is possible to suppress positional shifting of the front waist portion 20. Also, the texture can be made softer in the portion where the front waist portion 20 fits against the wearer's skin.

Also, the up-down-direction length of the region between the upper end 10a of the absorbent main body 10 and the upper end 11ea of the absorbent body 11 is longer than the up-down-direction length of the region between the lower end 24b of the stretchy sheet 24 and the string-like elastic member 23a located uppermost (i.e., the middle region CR). Due to the up-down-direction length of the upper end portion of the absorbent main body 10 that is not overlapped with the absorbent body 11 being longer than the up-down-direction length of the middle region CR, the upper end portion of the absorbent main body 10 is more likely to fit to the wearer's stomach region, and it is possible to suppress positional shifting of the absorbent main body 10.

### Variations

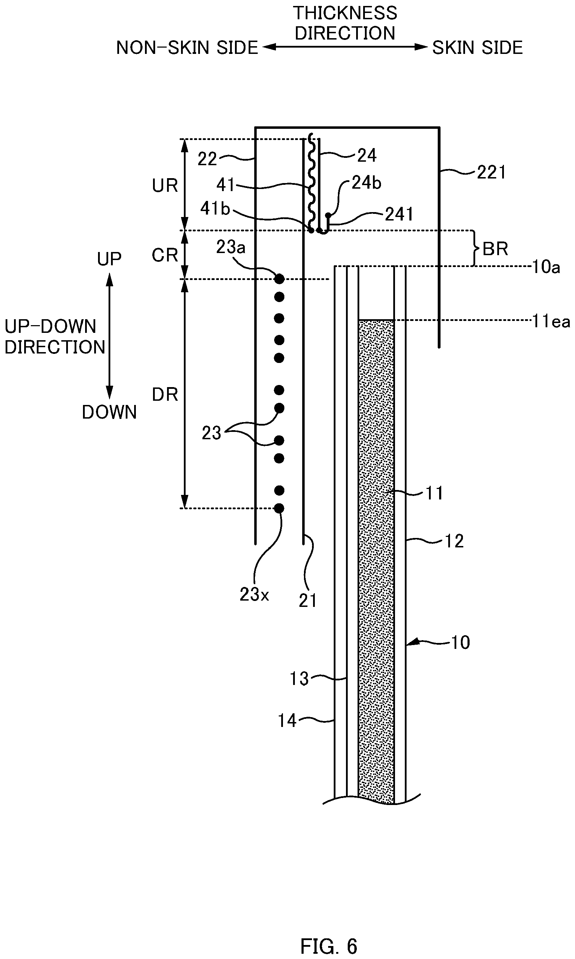

FIG. 6 is a schematic cross-sectional view illustrating a variation of a method for fixing the stretchy sheet 24 of the front waist portion 20. An adhesive such as a hot-melt adhesive is applied to a fixing portion 41, the fixing portion 41 is provided between the stretchy sheet 24 and the non-stretchy sheets (inner layer sheet 21 and outer layer sheet 22), and the stretchy sheet 24 is fixed to the non-stretchy sheets 21 and 22 by the fixing portion 41.

In the front waist portion 20 shown in FIGS. 3 and 4, the stretchy sheet 24 is arranged so as to extend straight without being folded in the up-down direction. In other words, the upward region UR is formed between the upper end 24a and the lower end 24b of the stretchy sheet 24. However, in the variation in FIG. 6, the lower end 24b of the stretchy sheet 24 extends downward beyond a lower end 41b of the fixing portion 41, and the extending portion is folded upward at the lower end 41b of the fixing portion 41, thus forming a folded portion 241. In other words, a portion of the stretchy sheet 24 that protrudes downward from the lower end 41b of the fixing portion 41 is turned upward by the contractive force of the stretchy sheet 24 itself, thus forming the folded portion 241.

In the region where the folded portion 241 is formed in the lower end portion of the stretchy sheet 24, the stretchy sheet 24 is overlaid on itself in the thickness direction, and therefore in this overlaid region, the stretchability of the stretchy sheet 24 increases locally, and the front waist portion 20 is likely to come into close contact with the wearer's skin. Accordingly, positional shifting of the front waist portion 20 is not likely to occur when the diaper 1 is put on.

Also, in the case where the folded portion 241 is formed, the above-described buffer region BR is the region between the folding position of the stretchy sheet 24 (i.e., the lower end 41b of the fixing portion 41) and the upper end 10a of the absorbent main body 10 in the up-down direction. Accordingly, the up-down-direction lengths (up-down-direction sizes) of the buffer region BR and the upward region UR can be adjusted by adjusting the position (height) at which the fixing portion 41 is provided. Accordingly, it is possible to provide a diaper with a buffer region BR having a size that is set appropriately according to the age and physique of the target wearer.

### Other embodiments

Although the embodiment of the present disclosure have been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof. For example, modification which will be described below is possible.

For example, the stretchy sheet 24 is not limited to being provided in the upper end portion of the front waist portion 20. The stretchy sheet 24 may be provided at any position in the front waist portion 20 in the up-down direction. Also, a configuration is possible in which the back waist portion 30 (first waist portion) has a stretchy sheet, and the front waist portion 20 (second waist portion) does not have a stretchy sheet, or the front waist portion 20 and the back waist portion 30 may both have the stretchy sheet 24.

Also, although a diaper in which the front waist portion 20 and the back waist portion 30 are separate members is described as an example in the above embodiment, a configuration is possible in which the crotch portion is provided between the front waist portion 20 and the back waist portion 30, and the front waist portion 20 and the back waist portion 30 are a single continuous member.

### [Reference Signs List]

1 diaper (underpants-shaped absorbent article), 2 joining region,
10 absorbent main body, 10a upper end,
11 absorbent body, 11ea upper end,
11A absorbent core, 11B core-wrapping sheet,
12 top sheet, 13 back sheet,
14 exterior sheet, 15 leg elastic member,
20 front waist portion (first waist portion),
21 inner layer sheet (non-stretchy sheet), 22 outer layer sheet (non-stretchy sheet),
23 string-like elastic member, 23a string-like elastic member, 23x string-like elastic member,
24 stretchy sheet, 24a upper end, 24b lower end,
241 folded portion,
30 back waist portion (second waist portion), 31 inner layer sheet,
32 outer layer sheet, 33 string-like elastic member,
41 fixing portion,
BR buffer region, UR upward region, CR middle region, DR downward region

## Claims

1. An underpants-shaped absorbent article having an up-down direction, a left-right direction, and a front-rear direction that intersect each other,
the underpants-shaped absorbent article comprising:
an absorbent main body (10); and
a first waist portion (20) that is joined to the absorbent main body (10) and that includes a stretchy sheet (24) having stretchability in the left-right direction,
the stretchy sheet (24) having a lower end (24b),
the lower end (24b) being located at a position separated upward in the up-down direction by a predetermined distance (1g) from an upper end (10a) of the absorbent main body (10), wherein
the first waist portion (20) includes a plurality of elastic members (23) having stretchability in the left-right direction, and
a left-right-direction mean stress (σ_{BR}) in an up-down-direction region (BR) of the first waist portion (20) that is between the lower end (24b) of the stretchy sheet (24) and an elastic member (23a) located at an uppermost position among the plurality of elastic members (23)
is smaller than
a left-right-direction mean stress (σ_{UR}) in an up-down-direction region (UR) of the first waist portion (20) in which the stretchy sheet (24) is arranged, and wherein
an up-down-direction length of the up-down-direction region (UR) in which the stretchy sheet (24) is arranged
is longer than
an up-down-direction length between the lower end (24b) of the stretchy sheet (24) and the elastic member (23a) located uppermost.

2. The underpants-shaped absorbent article according to claim 1, wherein
the elastic members (23) are arranged in at least a portion of an up-down-direction region (DR) of the first waist portion (20) that is overlapped with the absorbent main body (10).

3. The underpants-shaped absorbent article according to claim 2, wherein
the elastic members (23) are not arranged in an up-down-direction region (BR) of the first waist portion (20) in which the stretchy sheet (24) and the absorbent main body (10) are not overlapped.

4. The underpants-shaped absorbent article according to any one of claims 1 to 3, wherein
the first waist portion (20) includes a plurality of the elastic members (23), and
the left-right-direction mean stress (σ_{UR}) in the up-down-direction region (UR) of the first waist portion (20) in which the stretchy sheet (24) is arranged
is smaller than
a left-right-direction mean stress (σ_{DR}) in an up-down-direction region (DR) of the first waist portion (20) in which the plurality of elastic members (23) are arranged.

5. The underpants-shaped absorbent article according to any one of claims 1 to 4, wherein
the first waist portion (20) includes a plurality of the elastic members (23), and
concerning an up-down-direction region (DR) of the first waist portion (20) in which the plurality of elastic members (23) are arranged,
a left-right-direction mean stress (σ_{DR1}) in a region (DR1) that is above a center in the up-down direction is smaller than a left-right-direction mean stress (σ_{DR2}) in a region (DR2) that is below the center in the up-down direction.

6. The underpants-shaped absorbent article according to claim 5, wherein
the absorbent main body (10) includes an absorbent body (11) that absorbs an excreted fluid, and
a left-right-direction mean stress (σ_{DR3}) in an up-down-direction region (DR3) of the first waist portion (20) that is between the elastic member (23a) located uppermost and an upper end (11ea) of the absorbent body (11)
is smaller than
a left-right-direction mean stress (σ_{DR4}) in an up-down-direction region (DR4) of the first waist portion (20) that is between the upper end (11ae) of the absorbent body (11) and an up-down-direction center of the up-down-direction region (DR) in which the plurality of elastic members (23) are arranged.

7. The underpants-shaped absorbent article according to any one of claims 1 to 6, wherein
the absorbent main body (10) includes an absorbent body (11) that absorbs an excreted fluid,
the first waist portion (20) includes a plurality of the elastic members (23), and
an up-down-direction length between the upper end (10a) of the absorbent main body (10) and an upper end (11ae) of the absorbent body (11)
is longer than
an up-down-direction length between the lower end (24b) of the stretchy sheet (24) and the elastic member (23a) located uppermost.

8. The underpants-shaped absorbent article according to any one of claims 1 to 7, wherein
the underpants-shaped absorbent article further comprises:
a non-stretchy sheet (24) that constitutes the first waist portion (20); and
a fixing portion (41) that fixes the stretchy sheet (24) to the non-stretchy sheet (24),
the stretchy sheet (24) has a lower end portion in the up-down direction, and
the lower end portion of the stretchy sheet (24) includes a folded portion (241) that has been folded upward at a lower end (41b) of the fixing portion (241).

## Patentansprüche

1. Unterhosen-förmiger absorbierender Artikel, der eine Oben-Unten-Richtung, eine Links-Rechts-Richtung und eine Vorn-Hinten-Richtung aufweist, die einander schneiden,
wobei der Unterhosen-förmige absorbierende Artikel Folgendes umfasst:
einen absorbierenden Hauptkörper (10); und
einen ersten Taillenteil (20), der mit dem absorbierenden Hauptkörper (10) verbunden ist und der eine dehnbare Lage (24) einschließt, die eine Dehnbarkeit in der Links-Rechts-Richtung aufweist,
wobei die dehnbare Lage (24) ein unteres Ende (24b) aufweist,
sich das untere Ende (24b) an einer Position befindet, die nach oben in der Oben-Unten-Richtung durch einen vorgegebenen Abstand (1g) von einem oberen Ende (10a) des absorbierenden Hauptkörpers (10) getrennt ist, wobei
der erste Taillenteil (20) eine Vielzahl von elastischen Elementen (23) einschließt, die eine Dehnbarkeit in der Links-Rechts-Richtung aufweisen, und
eine Mittelspannung in der Links-Rechts-Richtung (σ_{BR}) in einem Bereich der Oben-Unten-Richtung (BR) des ersten Taillenteils (20), der zwischen dem unteren Ende (24b) der dehnbaren Lage (24) und einem elastischen Element (23a) vorliegt, das sich an einer obersten Position unter der Vielzahl von elastischen Elementen (23) befindet,
kleiner ist als
eine Mittelspannung in der Links-Rechts-Richtung (σ_{UR}) in einem Bereich der Oben-Unten-Richtung (UR) des ersten Taillenteils (20), in dem die dehnbare Lage (24) angeordnet ist, und wobei
eine Länge in der Oben-Unten-Richtung des Bereichs der Oben-Unten-Richtung (UR), in dem die dehnbare Lage (24) angeordnet ist,
länger ist als
eine Länge in der Oben-Unten-Richtung zwischen dem unteren Ende (24b) der dehnbaren Lage (24) und dem elastischen Element (23a), das sich ganz oben befindet.

2. Unterhosen-förmiger absorbierender Artikel nach Anspruch 1, wobei
die elastischen Elemente (23) in mindestens einem Teil eines Bereichs der Oben-Unten-Richtung (DR) des ersten Taillenteils (20) angeordnet sind, der mit dem absorbierenden Hauptkörper (10) überlappt ist.

3. Unterhosen-förmiger absorbierender Artikel nach Anspruch 2, wobei
die elastischen Elemente (23) nicht in einem Bereich der Oben-Unten-Richtung (BR) des ersten Taillenteils (20) angeordnet sind, in dem die dehnbare Lage (24) und der absorbierende Hauptkörper (10) nicht überlappt sind.

4. Unterhosen-förmiger absorbierender Artikel nach einem der Ansprüche 1 bis 3, wobei
der erste Taillenteil (20) eine Vielzahl der elastischen Elemente (23) einschließt und
die Mittelspannung in der Links-Rechts-Richtung (σ_{UR}) in dem Bereich der Oben-Unten-Richtung (UR) des ersten Taillenteils (20), in dem die dehnbare Lage (24) angeordnet ist,
kleiner ist als
eine Mittelspannung in der Links-Rechts-Richtung (σ_{DR}) in einem Bereich der Oben-Unten-Richtung (DR) des ersten Taillenteils (20), in dem die Vielzahl von elastischen Elementen (23) angeordnet ist.

5. Unterhosen-förmiger absorbierender Artikel nach einem der Ansprüche 1 bis 4, wobei
der erste Taillenteil (20) eine Vielzahl der elastischen Elemente (23) einschließt und
bezüglich eines Bereichs der Oben-Unten-Richtung (DR) des ersten Taillenteils (20), in dem die Vielzahl von elastischen Elementen (23) angeordnet ist,
eine Mittelspannung in der Links-Rechts-Richtung (σ_{DR1}) in einem Bereich (DR1), der über einer Mitte in der Oben-Unten-Richtung vorliegt, kleiner ist als eine Mittelspannung in der Links-Rechts-Richtung (σ_{DR2}) in einem Bereich (DR2), der unter der Mitte in der Oben-Unten-Richtung vorliegt.

6. Unterhosen-förmiger absorbierender Artikel nach Anspruch 5, wobei
der absorbierende Hauptkörper (10) einen Saugkörper (11) einschließt, der eine ausgeschiedene Flüssigkeit absorbiert, und
eine Mittelspannung in der Links-Rechts-Richtung (σ_{DR3}) in einem Bereich der Oben-Unten-Richtung (DR3) des ersten Taillenteils (20), der zwischen dem elastischen Element (23a), das sich ganz oben befindet, und einem oberen Ende (11ea) des Saugkörpers (11) vorliegt,
kleiner ist als
eine Mittelspannung in der Links-Rechts-Richtung (σ_{DR4}) in einem Bereich der Oben-Unten-Richtung (DR4) des ersten Taillenteils (20), der zwischen dem oberen Ende (11ea) des Saugkörpers (11) und einer Mitte in der Oben-Unten-Richtung des Bereichs der Oben-Unten-Richtung (DR), in dem die Vielzahl von elastischen Elementen (23) angeordnet ist, vorliegt.

7. Unterhosen-förmiger absorbierender Artikel nach einem der Ansprüche 1 bis 6, wobei
der absorbierende Hauptkörper (10) einen Saugkörper (11) einschließt, der eine ausgeschiedene Flüssigkeit absorbiert,
der erste Taillenteil (20) eine Vielzahl der elastischen Elemente (23) einschließt und
eine Länge in der Oben-Unten-Richtung zwischen dem oberen Ende (10a) des absorbierenden Hauptkörpers (10) und einem oberen Ende (11ae) des Saugkörpers (11)
länger ist als
eine Länge in der Oben-Unten-Richtung zwischen dem unteren Ende (24b) der dehnbaren Lage (24) und dem elastischen Element (23a), das sich ganz oben befindet.

8. Unterhosen-förmiger absorbierender Artikel nach einem der Ansprüche 1 bis 7, wobei
der Unterhosen-förmige absorbierende Artikel weiter Folgendes umfasst:
eine nicht-dehnbare Lage (24), die den ersten Taillenteil (20) bildet; und
einen Befestigungsteil (41), der die dehnbare Lage (24) mit der nichtdehnbaren Lage (24) befestigt,
die dehnbare Lage (24) einen unteren Endteil in der Oben-Unten-Richtung aufweist und
der untere Endteil der dehnbaren Lage (24) einen gefalteten Teil (241) einschließt, der nach oben an einem unteren Ende (41b) des Befestigungsteils (241) gefaltet wurde.

## Revendications

1. Article absorbant en forme de culotte ayant une direction de haut en bas, une direction de gauche à droite, une direction d'avant en arrière qui s'entrecroisent l'une l'autre,
l'article absorbant en forme de culotte comprenant :
un corps principal absorbant (10) ; et
une première partie de ceinture (20) qui est jointe au corps principal absorbant (10) et qui comprend une feuille extensible (24) ayant une extensibilité dans la direction de gauche à droite,
la feuille extensible (24) ayant une extrémité inférieure (24b),
l'extrémité inférieure (24b) étant située à une position séparée vers le haut dans la direction de haut en bas d'une distance prédéterminée (1g) d'une extrémité supérieure (10a) du corps principal absorbant (10), dans lequel
la première partie de ceinture (20) comprend une pluralité de membres élastiques (23) ayant une extensibilité dans la direction de gauche à droite, et
une contrainte moyenne de gauche à droite (σ_{BR}) dans une région dans la direction de haut en bas (BR) de la première partie de ceinture (20) qui est entre l'extrémité inférieure (24b) de la feuille extensible (24) et un membre élastique (23a) situé à une position la plus haute parmi la pluralité de membres élastiques (23), est plus basse qu'une contrainte moyenne de gauche à droite (σ_{UR}) dans une région dans la direction de haut en bas (UR) de la première partie de ceinture (20) dans laquelle la feuille extensible (24) est arrangée, et dans lequel
une longueur dans la direction de haut en bas de la région dans la direction de haut en bas (UR) dans laquelle la feuille extensible (24) est arrangée est plus longue qu'une longueur dans la direction de haut en bas entre l'extrémité inférieure (24b) de la feuille extensible (24) et le membre élastique (23a) situé le plus en haut.

2. Article absorbant en forme de culotte selon la revendication 1, dans lequel les membres élastiques (23) sont arrangés dans au moins une partie d'une région dans la direction de haut en bas (DR) de la première partie de ceinture (20) que le corps principal absorbant (10) chevauche.

3. Article absorbant en forme de culotte selon la revendication 2, dans lequel
les membres élastiques (23) ne sont pas arrangés dans une région dans la direction de haut en bas (BR) de la première partie de ceinture (20) dans laquelle la feuille extensible (24) et le corps principal absorbant (10) ne se chevauchent pas.

4. Article absorbant en forme de culotte selon l'une quelconque des revendications 1 à 3, dans lequel
la première partie de ceinture (20) comprend une pluralité de membres élastiques (23), et
la contrainte moyenne dans la direction de gauche à droite (σ_{UR}) dans la région dans la direction de haut en bas (UR) de la première partie de ceinture (20) dans laquelle la feuille extensible (24) est arrangée, est plus basse qu'une contrainte moyenne dans la direction de gauche à droite (σ_{DR}) dans une région dans la direction de haut en bas (DR) de la première partie de ceinture (20) dans laquelle la pluralité de membres élastiques (23) sont arrangés.

5. Article absorbant en forme de culotte selon l'une quelconque des revendications 1 à 4, dans lequel
la première partie de ceinture (20) comprend une pluralité de membres élastiques (23), et
au sujet d'une région dans la direction de haut en bas (DR) de la première partie de ceinture (20) dans laquelle la pluralité de membres élastiques (23) sont arrangés,
une contrainte moyenne dans la direction de gauche à droite (σ_{DR1}) dans une région (DR1) qui est au-dessus d'un centre dans la direction de haut en bas, est plus basse qu'une contrainte moyenne dans la direction de gauche à droite (σ_{DR2}) dans une région (DR2) qui est au-dessous du centre dans la direction de haut en bas.

6. Article absorbant en forme de culotte selon la revendication 5, dans lequel
le corps principal absorbant (10) comprend un corps absorbant (11) qui absorbe un fluide excrété, et
une contrainte moyenne dans la direction de gauche à droite (σ_{DR3}) dans une région dans la direction de haut en bas (DR3) de la première partie de ceinture (20) qui est entre le membre élastique (23a) situé le plus en haut et une extrémité supérieure (11ea) du corps absorbant (11), est plus basse qu'une contrainte moyenne dans la direction de gauche à droite (σ_{DR4}) dans une région dans la direction de haut en bas (DR4) de la première partie de ceinture (20) qui est entre l'extrémité supérieure (11ae) du corps absorbant (11) et un centre dans la direction de haut en bas de la région dans la direction de haut en bas (DR) dans laquelle la pluralité de membres élastiques (23) sont arrangés.

7. Article absorbant en forme de culotte selon l'une quelconque des revendications 1 à 6, dans lequel
le corps principal absorbant (10) comprend un corps absorbant (11) qui absorbe un fluide excrété,
la première partie de ceinture (20) comprend une pluralité de membres élastiques (23), et
une longueur dans la direction de haut en bas entre l'extrémité supérieure (10a) du corps principal absorbant (10) et une extrémité supérieure (11ae) du corps absorbant (11), est plus longue qu'une longueur dans la direction de haut en bas entre l'extrémité inférieure (24b) de la feuille extensible (24) et le membre élastique (23a) situé le plus en haut.

8. Article absorbant en forme de culotte selon l'une quelconque des revendications 1 à 7, où
l'article absorbant en forme de culotte comprend en outre :
une feuille non extensible (24) qui constitue la première partie de ceinture ; et
une partie de fixation (41) qui fixe la feuille extensible (24) à la feuille non extensible (24),
la feuille extensible (24) a une partie d'extrémité inférieure dans la direction de haut en bas, et
la partie d'extrémité inférieure de la feuille extensible (24) comprend une partie repliée (241) qui a été repliée vers le haut à une extrémité inférieure (41b) de la partie de fixation (241).
